# FASCICULE DE BREVET EUROPEEN

(11) **EP 3 681 595 B1**
(45) Date de publication et mention de la délivrance du brevet: **16.03.2022**
(21) Numéro de dépôt: 18765134.4
(22) Date de dépôt: 11.09.2018
(51) Int. Cl.: A61N 1/378

(54) **DISPOSITIF MOTEUR IMPLANTABLE ET DISPOSITIF ELECTROGENERATEUR IMPLANTABLE COMPORTANT UN TEL DISPOSITIF MOTEUR**
IMPLANTIERBARE MOTORVORRICHTUNG UND IMPLANTIERBARE ELEKTRISCHE GENERATORVORRICHTUNG, DIE EINE SOLCHE MOTORVORRICHTUNG UMFASST
IMPLANTABLE MOTOR DEVICE AND IMPLANTABLE ELECTRICITY-GENERATING DEVICE COMPRISING SUCH A MOTOR DEVICE

(30) Priorité: 13.09.2017 EP 17190836; 14.09.2017 CH 11392017
(43) Date de publication de la demande: 22.07.2020
(73) Titulaire: Freymond, Eric, London W1K 2RS (GB)
(72) Inventeur: RENAUD, M. Dominique, 1110 Morges (CH)
(74) Mandataire: Bovard SA Neuchâtel
(86) Numéro de dépôt international: PCT/EP2018/074494
(87) Numéro de publication internationale: WO 2019/053014

(56) Documents cités:
- WO-A2-2006/024868
- WO-A2-2007/109272
- US-A- 3 826 265
- US-A1- 2010 076 517
- JANPHUANG PATTANAPHONG ET AL: "Harvesting Energy From a Rotating Gear Using an AFM-Like MEMS Piezoelectric Frequency Up-Converting Energy Harvester", JOURNAL OF MICROELECTROMECHANICAL SYSTEMS, IEEE SERVICE CENTER, US, vol. 24, no. 3, 1 juin 2015 (2015-06-01), pages 742-754, XP011583325, ISSN: 1057-7157, DOI: 10.1109/JMEMS.2014.2349794 [extrait le 2015-06-01]

## Description

### Domaine technique

La présente invention, telle que définie dans la revendication 1, se rapporte au domaine des dispositifs médicaux biocompatibles. Elle concerne, plus particulièrement, un dispositif moteur implantable adapté pour capter, accumuler et stocker une quantité d'énergie biomécanique sous forme mécanique et pour redistribuer cette énergie mécanique de manière séquentielle à un dispositif consommateur.

L'invention concerne également un dispositif électrogénérateur comportant un dispositif moteur selon la revendication 1, accouplé à un générateur électrique pour générer une énergie électrique utile à l'alimentation d'un dispositif consommateur, notamment un implant médical.

### Etat de la technique

On connait depuis maintenant plus de 50 ans des dispositifs de stimulation électrique implantables dans le corps d'un patient, ce dernier pouvant être humain ou animal. L'exemple le plus emblématique de tels dispositifs est certainement le stimulateur cardiaque, ou « pacemaker » en anglais, conçu pour supporter le fonctionnement du muscle cardiaque chez des patients dont la fonction cardiaque est affectée.

Depuis la première implantation réussie d'un stimulateur cardiaque en 1958 ces dispositifs se sont démocratisés et développés, et de nombreux autres ont été proposés dans le but d'assister par stimulation électrique au fonctionnement de certains organes, ou encore de traiter par stimulation électrique ciblée certaines pathologies, notamment au niveau du système neuro-végétatif.

On connait par ailleurs un grand nombre de dispositifs médicaux implantables non destinés à la procuration d'une stimulation électrique d'un organe mais à l'assistance ou la palliation d'une fonction organique défaillante. On peut notamment citer à titre indicatif les implants cochléaires, ou encore les sphincters artificiels et pompes à insuline.

Tous ces dispositifs implantables ont pour point commun de requérir une alimentation en énergie électrique, continue ou ponctuelle, pour pouvoir fonctionner. Une telle énergie électrique est à ce jour fournie essentiellement par des batteries implantables ou, dans certains cas, des batteries exogènes, positionnées dans un boitier externe au corps d'un patient et raccordées par des connectiques appropriées au dispositif médical implantable.

Ces batteries ont toutefois selon leur taille, leur nature mais aussi la nature et le mode de fonctionnement du dispositif médical implanté qu'elles alimentent, des durées de vie limitées, qui nécessitent le cas échéant un remplacement ou un rechargement. Lorsque les batteries sont exogènes cela ne pose bien entendu pas de difficultés particulières. En revanche, lorsque les batteries sont implantées, leur remplacement nécessite une intervention chirurgicale du patient avec les désagréments associés. Dans certains cas, il est possible d'opérer le rechargement des batteries implantées sans intervention invasive pour le patient, par des moyens ambulatoires de rechargement par induction.

Toutefois, de tels moyens de rechargement ne peuvent être employés que lorsque les implants sont relativement proches de la surface cutanée du patient. Pour pallier cette limitation il est possible dans certains cas de déporter la batterie de l'implant par une connexion filaire, la batterie étant alors disposée en sous-cutané dans le corps du patient, par exemple au niveau de l'abdomen. Toutefois une telle connexion déportée implique des risques de disfonctionnement liés à des défauts de connexion électrique entre la batterie et l'implant ainsi qu'à des problèmes d'étanchéité et de corrosion.

Un générateur électromécanique implantable a été proposé dans la demande de brevet WO 2006/024868 A1, qui décrit un dispositif selon le préambule de la revendication 1. . Celui-ci repose sur la captation d'une énergie corporelle liée aux mouvements relatifs d'organes ou membres d'un patient accumulée sous forme d'énergie mécanique dans un ressort en spirale. L'énergie mécanique accumulée dans le ressort peut ensuite être transmise sous forme de couple et d'une transmission par détente du ressort une fois que celui-ci a été complètement bandé à une génératrice électrique de type alternateur. Un inconvénient cependant de cette solution réside dans le fait que l'énergie du ressort n'est transmise à la génératrice que de façon irrégulière quand le ressort est complètement armé, ce qui implique une production d'énergie électrique irrégulière non propice à une utilisation directe par un dispositif consommateur implanté ou nécessitant une phase de stockage préalable importante pour permettre une utilisation fiable du dispositif consommateur.

Vu les inconvénients susvisés des dispositifs médicaux requérant une alimentation électrique pour leur bon fonctionnement, la présente invention a pour but de proposer un dispositif électro-générateur implantable d'un genre nouveau, qui permette de simplifier notamment le rechargement de batteries d'alimentation de tels dispositifs, avec une grande fiabilité de fonctionnement et qui soit indépendant de toute source d'énergie externe au patient.

### Divulguation de l'invention

L'invention a ainsi pour objet, selon la revendication 1, un dispositif moteur implantable comportant une source d'énergie mécanique agencée mobile en rotation autour d'un arbre définissant un axe de rotation et en liaison cinématique avec, d'une part, un dispositif de chargement de la source d'énergie mécanique, et, d'autre part, un organe d'accouplement à un dispositif consommateur d'énergie. Le dispositif de chargement de la source d'énergie mécanique comporte avantageusement un premier et un second organes de fixation respectivement à un premier et un second membres ou organes mobiles l'un par rapport à l'autre dans le corps d'un patient, agencés tel qu'un déplacement relatif des premier et second membres ou organes entraine un déplacement relatif analogue desdits premier et second organes de fixation. Il comporte également des moyens de remontage de la source d'énergie mécanique. Les moyens de remontage sont aptes à être connectés aux dits premier et second organes de fixation et comportent :
i. un rochet solidaire de l'arbre de rotation, ledit rochet comportant une denture périphérique triangulaire, et
ii. un volant d'entrainement unidirectionnel du rochet en rotation avec l'arbre, ledit volant portant des cliquets montés mobiles en rotation sur une serge du volant lesdits cliquets comportant une denture complémentaire de celle du rochet de manière à engrener avec la denture périphérique du rochet dans une sens de rotation uniquement lors de chaque déplacement relatif des dits premier et second organes de fixation.

De manière caractéristique suivant l'invention le dispositif moteur implantable proposé comporte en outre des moyens de libération séquentielle desdits moyens de remontage de la source d'énergie pour libérer cette dernière en rotation afin de transmettre un couple d'entrainement disponible à l'organe d'accouplement. Ces moyens de libération séquentielle desdits moyens de remontage de la source d'énergie mécanique comportent avantageusement :
- une came solidaire de la source d'énergie mécanique et
- un cliquet de déclenchement monté mobile en rotation autour d'un axe parallèle à l'axe de rotation de l'arbre de la source d'énergie, une extrémité dudit cliquet étant maintenue en appui par un organe de rappel dans une encoche formée dans une surface de ladite came, et
- un doigt de déclenchement solidaire dudit cliquet de déclenchement et agencé pour coopérer avec une goupille solidaire du rochet dans une position unique dudit rochet de manière à pivoter ledit cliquet de déclenchement de telle sorte que son extrémité sorte de l'encoche de la came et libère la source d'énergie mécanique en rotation pour transmettre un couple d'entrainement à l'organe d'accouplement,
- un levier de blocage solidaire en rotation du cliquet de déclenchement et agencé pour coopérer en butée avec un doigt de blocage lorsque l'extrémité du cliquet de déclenchement est insérée dans l'encoche. Le dispositif moteur implantable de la présente invention présente ainsi l'avantage essentiel de faire usage de l'énergie motrice biomécanique d'un patient, découlant des déplacements relatifs d'organes ou membres du squelette par exemple, pour déplacer les organes de fixation et ainsi charger de manière progressive le mécanisme d'accumulation et libération séquentielle d'énergie mécanique, lequel peut ensuite restituer cette énergie accumulée de manière régulière par son organe d'accouplement à tout dispositif consommateur, et notamment un générateur électrique selon un autre objet de la présente invention.

Le mécanisme d'accumulation et de libération séquentielle d'énergie s'avère particulièrement utile en ce qu'il permet d'accumuler de l'énergie biomécanique, même si les déplacements relatifs des organes de fixations et donc des membres auxquels ils sont fixés sont très faibles. De plus, lesmoyens de remontage permettent un remontage de la source d'énergie de façon fiable et sécurisée, et présentent de plus l'avantage de ne pas interférer avec la libération d'énergie accumulée par cette source.

Il est ainsi possible de s'affranchir des contraintes de conception et d'implantation de dispositifs générateurs biomécaniques reposant sur la mise en œuvre de sondes piezoélectriques pour récupérer l'énergie biomécanique dans le corps d'un patient.

Dans une forme de réalisation, la source d'énergie mécanique est préférentiellement un barillet, doté d'une cage munie d'une denture périphérique externe d'une part et logeant un ressort de barillet d'autre part, ledit ressort étant solidaire d'une paroi interne de la cage en une première extrémité et de l'arbre de rotation en une seconde extrémité.

L'utilisation d'un barillet permet l'accumulation d'énergie mécanique de manière très controllée et progressive via son ressort, ainsi que la libération de cette énergie de manière analogue, pour transmettre un couple souhaité en utilisant des moyens mécaniques de transmission très simples et facilement miniaturisables pour réduire l'encombrement du dispositif inventif.

Dans une forme particulière de réalisation, le rochet est monté libre en rotation par rapport au volant par l'intermédiaire d'un roulement à billes.

De façon avantageuse, les moyens de remontage comportent par ailleurs des cliquets de blocage du rochet, lesdits cliquets de blocage comportant une denture complémentaire de celle du rochet configurée pour empêcher une rotation du rochet en sens opposé à celui d'entrainement par le volant.

Selon une forme de réalisation avantageuse, un train de rouages de transmission est agencé en prise entre le mécanisme d'accumulation et de libération séquentielle d'énergie mécanique et l'organe d'accouplement à un dispositif consommateur.

Préférentiellement, le train de rouages est un train multiplicateur.

Dans une forme de réalisation particulière, le dispositif de l'invention comporte également un doigt de blocage des moyens de libération séquentielle est agencé pour coopérer en butée avec le levier de blocage solidaire du cliquet de déclenchement lorsque ce dernier est inséré dans l'encoche de la came.

Selon cette forme de réalisation, le levier du cliquet de dégagement comporte un profil de dégagement du doigt de blocage, agencé permettre une libération sensiblement instantanée du barillet en rotation dès la sortie du cliquet de déclenchement de l'encoche de la came lors de la levée du doigt de déclenchement par la goupille.

Dans une forme de réalisation du dispositif de l'invention l'organe d'accouplement est choisi parmi un mobile denté, une prise de force, un mobile d'entrainement magnétique.

Par ailleurs, selon une forme privilégiée de réalisation le mécanisme d'accumulation et de libération séquentielle d'énergie est agencé dans un boitier inoxydable et étanche, par exemple un carter en titane ou en alliages métallique de grade médical requis.

Selon un second aspect, l'invention concerne également un dispositif électrogénérateur implantable, comportant un dispositif moteur implantable tel que précédemment décrit et un générateur électromécanique comportant un rotor accouplé à l'organe d'accouplement du dispositif moteur.

Le rotor du générateur requérant une vitesse d'entrainement minimale pour permettre la génération d'un courant utile exploitable le mécanisme d'accumulation et libération séquentielle d'énergie mécanique du dispositif moteur de l'invention accumule ainsi l'énergie mécanique suffisante à l'entrainement à une vitesse souhaitée du rotor avant de la libérer et transmettre au rotor de manière régulière. L'énergie électrique générée alors peut être transmise à un dispositif implanté consommateur ou, de préférence, à une batterie d'accumulateurs qui est ainsi chargée de manière séquentielle par le dispositif moteur de l'invention.

Dans une forme de réalisation, le dispositif électro-générateur implantable comporte en outre un dispositif d'accumulation d'énergie électrique générée par le générateur électromécanique, électriquement connecté à une sortie dudit générateur.

Dans une forme de réalisation, le dispositif électro-générateur implantable de l'invention en outre des moyens électroniques de gestion de la distribution du courant délivré par le générateur.

Selon une forme de réalisation, le générateur électromécanique et le dispositif moteur d'entrainement sont agencés dans un boitier étanche.

De préférence, le générateur électromécanique est de type alternateur.

Enfin, pour permettre la connexion du dispositif de l'invention à un dispositif electro-consommateur, le dispositif de l'invention comporte également des moyens de connexion électrique étanche qui sont agencés sur le boitier.

### Brève description des dessins

D'autres détails de l'invention apparaîtront plus clairement à la lecture de la description qui suit, faite en référence aux dessins annexés dans lesquels :
- la figure 1 représente en vue de dessus le dispositif électrogénérateur de la présente invention dans un mode de réalisation,
- la figure 2 est une vue analogue à la figure 1, sans effet de transparence des éléments structurels superposés du dispositif,
- les figures 3 à 9 représentent la cinématique de fonctionnement du dispositif représenté aux figures 1 et 2 pour emmagasiner une énergie biomécanique et la restituer séquentiellement au générateur électromécanique du dispositif de l'invention pour générer un courant exploitable,
- la figure 10 représente un agrandissement permettant de visualiser l'ajustement de la goupille de déclenchement au doigt de déclenchement du dispositif d'accumulation et de libération séquentielle d'énergie mécanique du dispositif de l'invention,
- La figure 11 représente un mode de réalisation alternatif d'un disposif moteur implantable non couvert par les revendications, équipé d'un mécanisme de remontage inertiel de la source d'énergie ;
- Les figures 12 et 13 représentent respectivement des agrandissements des entrée et sortie du mécanisme d'accumulation et libération séquentielle d'énergie du dispositif moteur implantable de la figure 11.

### Mode de réalisation de l'invention

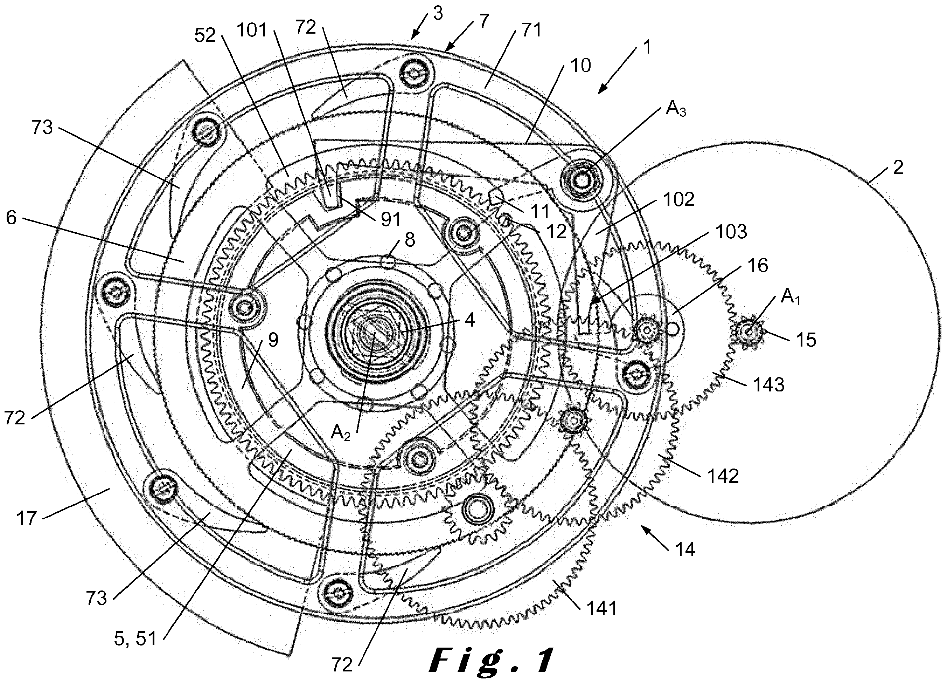

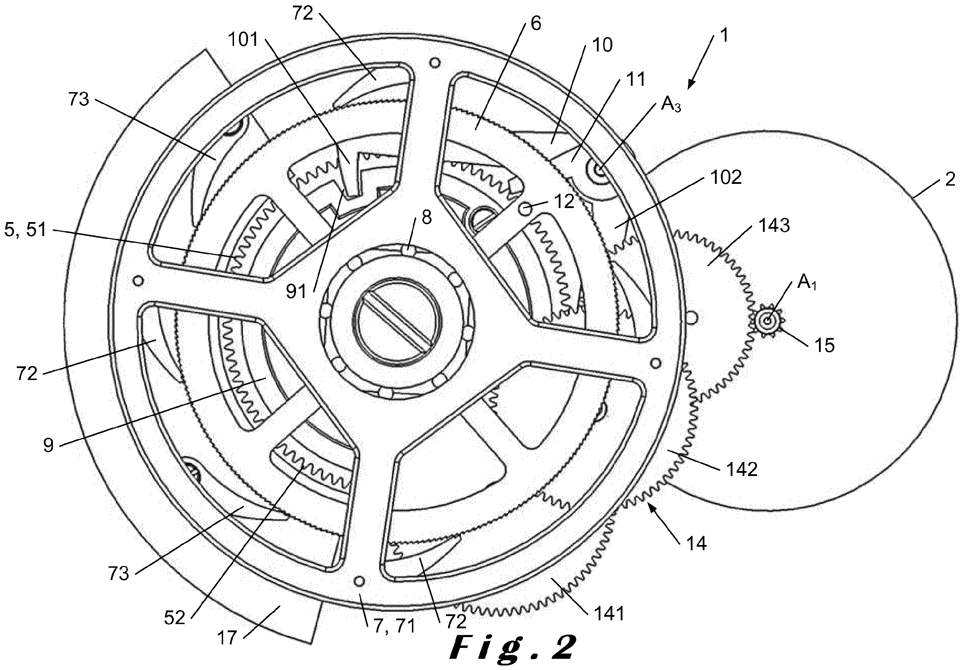

On a représenté sur les figures 1 et 2 un dispositif électro-générateur implantable 1 conçu pour permettre l'alimentation d'implants médicaux électro-pilotés et actionnés, tels que électrostimulateurs cardiaques, sphincters artificiels ou autres.

Par opposition aux batteries remplacables ou rechargeables d'alimentation classiquement utilisées pour procurer l'énergie électrique requise au fonctionnement de tels dispositifs médicaux, le dispositif électro-générateur implantable 1 est configuré pour permettre une génération de courant électrique utile auto entretenue par conversion d'énergie biomécanique d'un patient implanté, issue des déplacements relatifs d'os du squelette ou d'organes ou membres du patient dans son quotidien, en énergie électrique exploitable.

Le dispositif électro-générateur implantable 1 de l'invention permet donc de s'affranchir du problème de durée de vie limitée des batteries d'alimentation actuelles pour assurer le fonctionnement d'implants médicaux sur des durées de plusieurs dizaines d'années.

Le dispositif électrogénérateur implantable 1 comporte un générateur électromécanique 2, notamment de type alternateur, comportant de façon commune un rotor, mobile en rotation sur un axe A1, et un stator, ainsi qu'un circuit électrique associé, relié mécaniquement, conformément à la présente invention, à un dispositif moteur implantable d'entrainement du rotor, décrit ci-après. Le dispositif moteur implantable de l'invention permet d'induire une rotation du rotor du générateur et la génération d'un courant électrique exploitable en sortie de stator et donc de générateur. Ce type de générateur électromécanique étant courant dans le commerce et connu dans l'état de la technique il n'en est pas donné plus de détail particulier à ce stade dans la présente description ni les dessins.

De manière caractéristique, le dispositif moteur implantable comporte tout d'abord un premier et un second organes de fixation (non représentés), respectivement à un premier et un second membres ou organes d'un patient. Ces dits premier et second organes de fixation ont pour fonction de conférer un premier et un second points d'ancrage dans deux lieux de l'organisme du patient présentant une mobilité relative, notamment et de manière non exclusive, au niveau de la cage thoracique, sur les cotes ou leur ancrage (sternum, colonne vertébrale).

Les premier et second organes de fixation sont par ailleurs agencés mobiles l'un par rapport à l'autre, de telle sorte qu'un déplacement relatif des membres auxquels ils sont fixés entraine un déplacement relatif analogue desdits premier et second organes de fixation.

De manière complémentaire, un mécanisme 3 d'accumulation et de libération séquentielle d'énergie mécanique est agencé en liaison cinématique avec les premiers et second organes de fixation d'une part et avec le rotor du générateur électromécanique 2 d'autre part. Ce mécanisme 3 d'accumulation et de libération séquentielle d'énergie mécanique est représenté en détail aux figures 1 et 2 en particulier.

Ce mécanisme 3 d'accumulation et de libération séquentielle d'énergie mécanique comporte dans le mode de réalisation présenté un barillet 5 comme source motrice d'accumulation et libération d'énergie mécanique. Un tel barillet 5 présente ainsi de préférence une structure analogue aux barillets utilisés dans le domaine de l'horlogerie. Le barillet 5 comporte ainsi une cage 51 munie d'un denture périphérique externe 52 et à l'intérieur de laquelle est logé un ressort de barillet non représenté sur les figures par souci de clarté. Le barillet 5 est monté pivotant autour d'un arbre 4 définissant un axe de rotation A2, parallèle à l'axe de rotation du rotor A1 du générateur.

Le ressort de barillet est agencé, de façon classique en soit, solidaire d'une paroi interne de la cage 51 en une première extrémité et de l'arbre de rotation 4 en une seconde extrémité.

Ainsi, une rotation de l'arbre 4 autour de l'axe A2 lorsque la cage 51 est fixe permet un enroulement du ressort autour dudit arbre 4 et un remontage du ressort de barillet et ainsi l'emmagasinement d'une énergie mécanique sous forme élastique dans le ressort, qui peut être transmise sous la forme d'un couple moteur via la denture périphérique 52 du barillet au rotor du générateur 2 par toute transmission idoine lorsque la cage de barillet 51 est libérée en rotation alors que l'arbre 4 est lui bloqué.

Pour permettre le remontage du ressort de barillet 5 via l'arbre 4 le mécanisme 3 d'accumulation et de libération séquentielle d'énergie mécanique comporte en outre des moyens de remontage connectés aux dits premier et second organes de fixation afin d'être en prise directe avec les organes du corps du patient dont la mobilité est source d'énergie de remontage du barillet.

Ces moyens de remontage comportent en premier lieu un rochet 6 muni d'une denture périphérique en dent de scie. Le rochet 6 est monté sur l'arbre 4, de section carré, de telle sorte qu'une rotation du rochet 6 ou de l'arbre 4 autour de l'axe A2 entraine la rotation de l'autre, ainsi que l'enroulement du ressort de barillet.

De préférence, les premier et second organes de fixation sont montés mobiles en rotation autour de l'axe A2 et un des premier ou second organes de fixation est agencé en lien cinématique avec l'arbre 4. Ainsi, tout déplacement relatif des premier et second organes de fixation entraine la rotation de l'arbre 4 autour de l'axe A2, et la rotation analogue du rochet 6.

Le mécanisme 3 comporte également un volant 7 d'entrainement du rochet 6 monté en rotation autour de l'axe A2 sur l'arbre 4. Ce volant 7 est le mobile d'entrée du mécanisme 3, reliée à un au moins des premier et second organes de fixation pour transmettre une énergie biomécanique au ressort de barillet. Le volant 7 porte sur sa serge 71 des cliquets 72 montés mobiles en rotation sur ladite serge 71. Ces cliquets 72 comportent en leur extrémité libre des dents complémentaires de la denture du rochet 6 de manière à coopérer avec la denture périphérique du rochet 6 pour entrainer ce dernier et l'arbre en rotation 4 dans le sens d'enroulement du ressort de barillet après chaque déplacement relatif des dits premier et second organes de fixation.

Afin d'éviter le retour en arrière du rochet 6 sous la force du ressort de barillet des crochets fixes 73 de blocage du rochet 6 sont agencés sur un châssis, par exemple formé dans l'exemple représenté d'un boitier 17 recevant un dispositif électronique d'accumulation et d'alimentation de l'énergie électrique produite par le générateur 2 par exemple. Toutefois tout autre support ou platine fixe par rapport aux éléments tournants du mécanisme 3 et du générateur 2 pourrait également former un châssis de montage des cliquets de blocage 73.

Avantageusement, les cliquets 72 de remontage du rochet 6 présentent à leur extrémité libre une denture d'un pas égal au pas de denture du rochet 6. Toutefois, les cliquets 72 sont disposés sur la serge 71 du volant 7 de manière non symétrique en rotation par rapport à l'axe A2 de rotation du volant, de telle sorte qu'il existe entre eux une différence d'un demi-pas de dent. En variante, la denture d'extrémité des cliquets 72 pourrait également être telle qu'elle soit égale à un demi pas de denture du rochet. Ceci permet avantageusement de garantir que toute rotation du volant 7, quelque soit sont amplitude (pour autant que celle-ci soit supérieure au demi-pas de denture précité) soit utile à l'entrainement du rochet et donc au remontage du ressort de barillet, même si celle-ci est d'amplitude très faible.

Le volant 7 est dans l'exemple représenté monté sur le rochet 6 par l'intermédiaire d'un roulement à billes 8 garantissant la liberté de mouvement de l'arbre 4 et du rochet 6 par rapport au volant 7 tout en offrant un encombrement minimal en épaisseur.

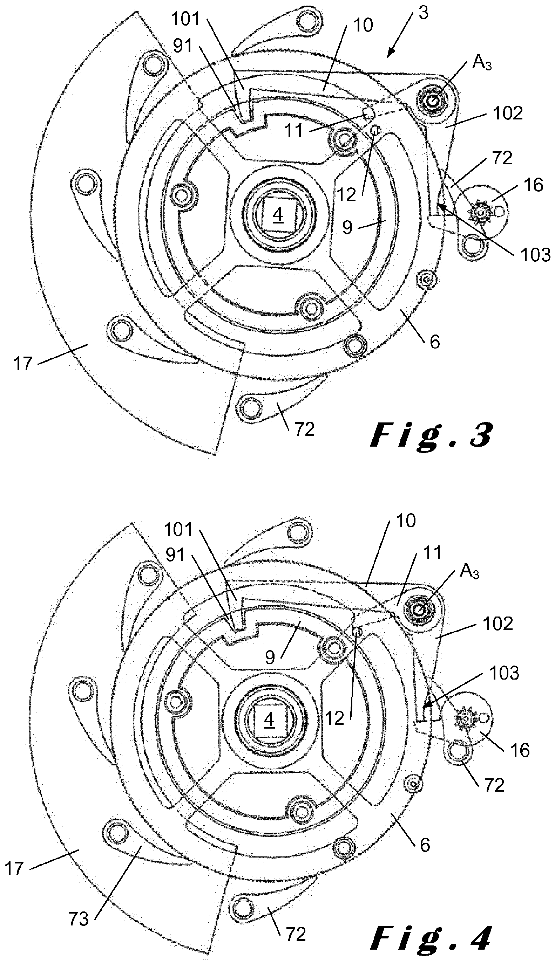

On comprend ainsi que toute rotation du volant 7 lors d'un déplacement relatif des organes de fixation entraine une rotation idoine du rochet 6 et de l'arbre 4 et l'enroulement du ressort de barillet par l'effet des cliquets 72, le rochet 6 se trouvant bloqué en rotation dans le sens opposé par l'intermédiaire des cliquets de blocage 73. Ainsi, le ressort de barillet peut être remonté et emmagasiner progressivement, pas à pas, l'énergie nécessaire au lancement du rotor du générateur 2. Cette phase de remontage est représentée aux figures 3 et 4.

Afin de libérer l'énergie emmagasinée par le barillet 5 le mécanisme 3 comporte en outre des moyens de libération séquentielle desdits moyens de remontage du barillet 5. On entend ici par libération séquentielle des moyens de remontage que le barillet 5 est libéré en rotation pour transmettre l'énergie mécanique emmagasinée par son ressort suivant une fréquence déterminée ou en une ou plusieurs position(s) déterminée(s) du rochet 6.

Dans l'exemple représenté, lesdits moyens de libération séquentielle permettent un déclenchement en une position déterminée du rochet 6 en rotation autour de l'axe A2. Lesdits moyens de libération séquentielle comportent :
- une came 9 solidaire du barillet 5 et
- un cliquet de déclenchement 10 monté mobile en rotation autour d'un axe A3 parallèle à l'axe A2 de rotation du barillet 5 et du rochet 6.

La came 9 est constituée dans l'exemple présenté d'un anneau de rayon constant autour de l'axe A2 sauf en une position dans laquelle la came 9 comprend un créneau ou encoche 91 dans laquelle est reçue une extrémité 101 dudit cliquet 10, lequel est maintenu en appui dans ladite encoche par un organe de rappel tel qu'un ressort de torsion logé dans un alésage de montage du cliquet sur son axe A3, par exemple formé d'une goupille.

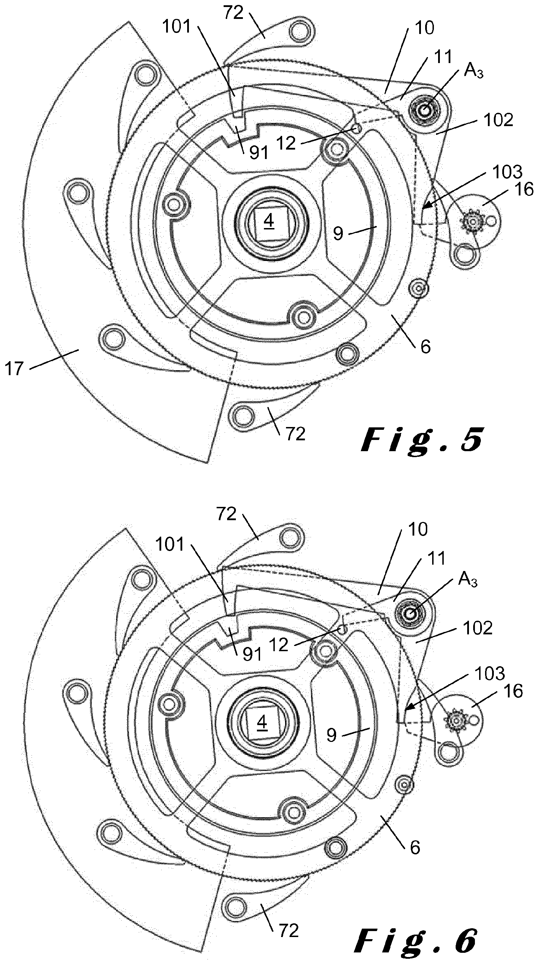

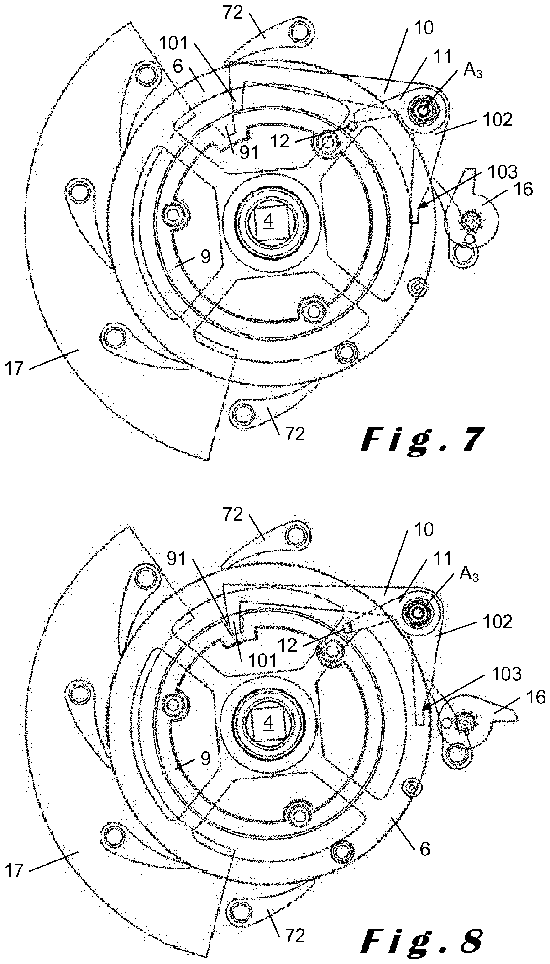

Comme cela ressort notamment des figures 5 à 7, un doigt de déclenchement 11 solidaire dudit cliquet de déclenchement 10 est agencé pour coopérer avec une goupille 12 fixée sur le rochet 6 de telle sorte que celle-ci engage l'extrémité du doigt de déclenchement 11 dans une position unique dudit rochet 6 de manière à pivoter ledit cliquet de déclenchement 10 et lever son extrémité 101 hors de l'encoche 91 de la came 9.

Les moyens de libération séquentielle comporte par ailleurs un levier de blocage 102 pivotant solidairement au cliquet de déclenchement 10 autour de l'axe A3. Ce levier de blocage 102 est agencé pour coopérer en butée sur son extrémité libre, opposée à son axe de rotation A3, avec un doigt 16 de blocage solidaire dans l'exemple présenté de l'axe de rotation du dernier mobile 143 d'un train de rouages 14 qui sera décrit par la suite. Le levier de blocage 102 et le doigt de blocage 16 sont agencés respectivement l'un par rapport à l'autre pour que l'extrémité du levier 102 coopère en appui plan, sans glissement, contre une surface plane opposée du doigt de blocage 16 lorsque le cliquet de déclenchement 10 est inséré dans l'encoche 91 de la came 9.

Le levier de blocage 102 pivote simultanément au cliquet de déclenchement 10 lorsque la goupille 12 engage le doigt de déclenchement 11, ce qui libère le doigt de blocage 16 en rotation par effet de liaison cinématique le barillet 5 via le train de rouage 14. Le barillet 5 et le train de rouages 14 poursuivent leur rotation durant une période T déterminée, choisie pour permettre la libération de l'énergie emmagasinée par son ressort depuis la libération précédente. Le maintien durant cette période T est assuré par le maintien en appui de l'extrémité 101 du cliquet 10 sur la surface de came 9, jusqu'à ce qu'elle retombe dans l'encoche 91.

La retombée du cliquet 10 dans l'encoche 91 provoque le retour du levier de blocage 102 en opposition du doigt de blocage 16, ce qui bloque à nouveau le barillet 5 pour un nouveau cycle d'emmagasinement de l'énergie.

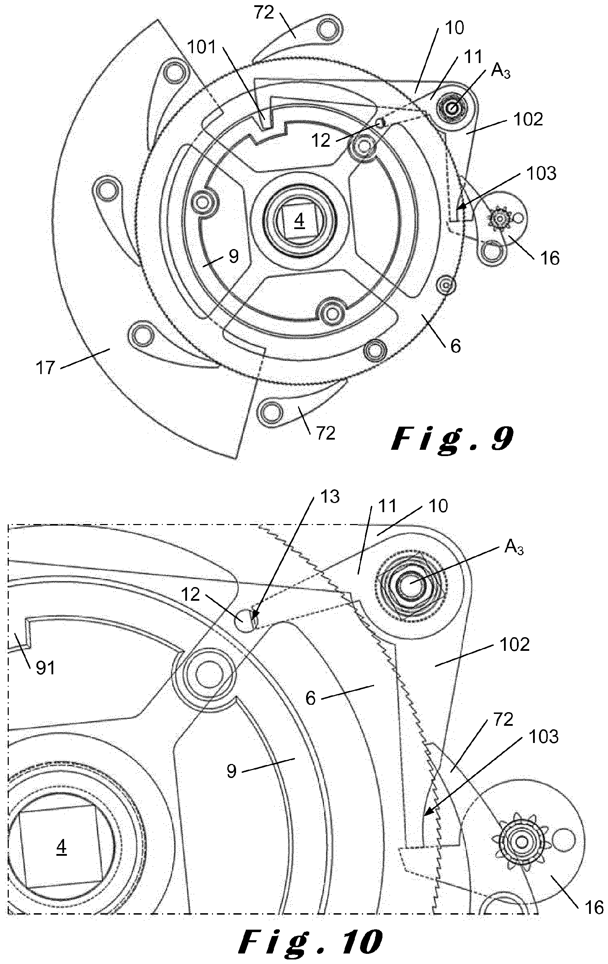

De manière complémentaire comme représenté aux figures 9 et 10, il est avantageux que la goupille 12 soit dotée d'un méplat 13 permettant éviter l'arcboutement de l'extrémité du doigt de déclenchement 11 contre la goupille 12 lors de la retombée du cliquet 10 dans l'encoche 91 de la came.

Avantageusement, le levier 102 du cliquet de dégagement 10 comporte un profil 103 de dégagement du doigt 16 de blocage du train de rouages 14, agencé pour permettre une libération sensiblement instantanée du barillet 5 et du train de rouages 14 en rotation dès la sortie du cliquet de déclenchement 10 de l'encoche de la came 9 lors de la levée du doigt de déclenchement 11 par la goupille 12. Ce profil 103 est nécessaire pour éviter le frottement du doigt de blocage 16 contre le levier 102, la vitesse de rotation du doigt de blocage 16 étant supérieure à celle du barillet par effet du train de rouage multiplicateur 14.

Lorsque le barillet 5 effectue sa rotation complète il transmet l'énergie préalablement emmagasinée sous la forme d'un couple d'entrainement au générateur 2 par sa denture 52 et par l'intermédiaire d'une transmission et d'un organe d'accouplement au rotor dudit générateurs idoines.

La transmission et l'organe d'accouplement peuvent par exemple comporter un train de rouages 14 multiplicateur composé d'une pluralité de mobiles dentés 141, 142, 143 et pignons associés, le premier mobile denté 141 du train 14 engrenant avec la denture 52 du barillet et le dernier mobile 143, qui forme alors organe d'accouplement du mécanisme 3, engrenant avec un pignon 15 solidaire du rotor du générateur 2. Par l'effet multiplicateur du train de rouages 14, il est ainsi possible d'entrainer le rotor autour de l'axe A1 à une vitesse beaucoup plus importante que la vitesse de rotation du barillet 5 autour de l'axe A2 tout en transmettant tout le couple moteur du barillet 5. Le générateur 2 peut alors générer un courant électrique utile, qui peut être exploité directement en sortie d'induit (stator) ou de préférence accumulé, traité et régulé en alimentation par un dispositif électronique 17 d'accumulation et de distribution vers un dispositif consommateur. Ce dispositif électronique 17 peut être logé dans le boitier 171 et doté avantageusement de moyens de communication sans fil pour être programmé à distance en fonction des paramètres d'alimentation requis pour un dispositif consommateur, notamment un implant paliatif au fonctionnement d'organes dans la corps d'un patient.

Il convient de noter ici que le mécanisme 3 du dispositif moteur de l'invention peut comporter tout autre type d'organe d'accouplement et pas uniquement un mobile denté comme le mobile 143 du train de rouage 14 dans le présent exemple. En particulier, une prise de force ou encore un module d'accouplement magnétique peuvent être agencés en liaison cinématique avec le train de rouage 14 pour transmettre le couple mécanique du barillet à un dispositif consommateur tel que le générateur 2.

De manière complémentaire et accessoire, le dispositif moteur et/ou le dispositif électrogénérateur 1 de l'invention peuvent comporter un automate de déclenchement des moyens de libération séquentielle en fonction d'une consigne externe, notamment par exemple un signal d'urgence transmis par un capteur localisé au niveau de l'organe d'accouplement ou d'un implant alimenté par le dispositif électrogénérateur 1. Un tel automate de déclenchement est avantageusement conçu et agencé pour pivoter le cliquet de déclenchement 10 et le levier de blocage 102 indépendamment de la position de la goupille 12 par rapport au doigt 11 en réponse à la consigne reçue et ainsi libérer le barillet 5 et le train de rouage 14.

Pour garantir l'implantabilité et la qualité médical requise au dispositif moteur de l'invention ainsi que le dispositif electrogénérateur 1 le comportant l'ensemble des éléments constitutifs de ces derniers notamment son générateur électromécanique 2 et le mécanisme 3 d'accumulation et de libération séquentielle d'énergie sont agencés dans un boitier inoxydable et étanche, de préférence en titane ou métal analogue très peu sensible à la corrosion et des moyens de connexion électrique étanche sont agencés sur ledit boitier et connectés à l'intérieur de celui-ci au dispositif électronique 17 afin de permettre l'alimentation d'un dit dispositif consommateur.

De façon complémentaire le boitier étanche comporte également des moyens de liaison mécanique étanche aux organes de fixations formant les moyens d'entrée du dispositif de l'invention pour récupérer l'énergie biomécanique entre deux organes/membres mobiles dans le corps d'un patient.

L'étanchéité des moyens de connexion électriques et mécaniques pourra en particulier être réalisée par revêtement du boitier à l'aide d'un emballage hermétique et bio-compatible constitué d'un complexe multi-couche SiOx / Parylene-C tel que développé par la société Coat-X SA.

L'invention procure ainsi un dispositif moteur implantable de nouvelle génération, permettant notamment d'alimenter un générateur pour produire une alimentation électrique d'un implant consommateur tel que pacemaker ou sphincter artificiel par exemple de longue durée, sans nécessité de rechargement ou de remplacement de batteries par le patient, le dispositif de l'invention permettant la génération d'un courant électrique par récupération in-situ d'énergie biomécanique dans le corps du patient entre des organes ou membres en mouvement de celui-ci, même de faible amplitude.

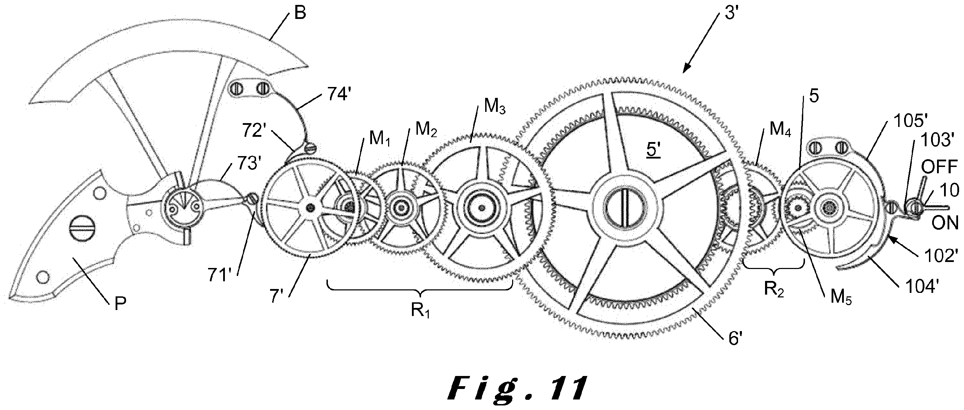

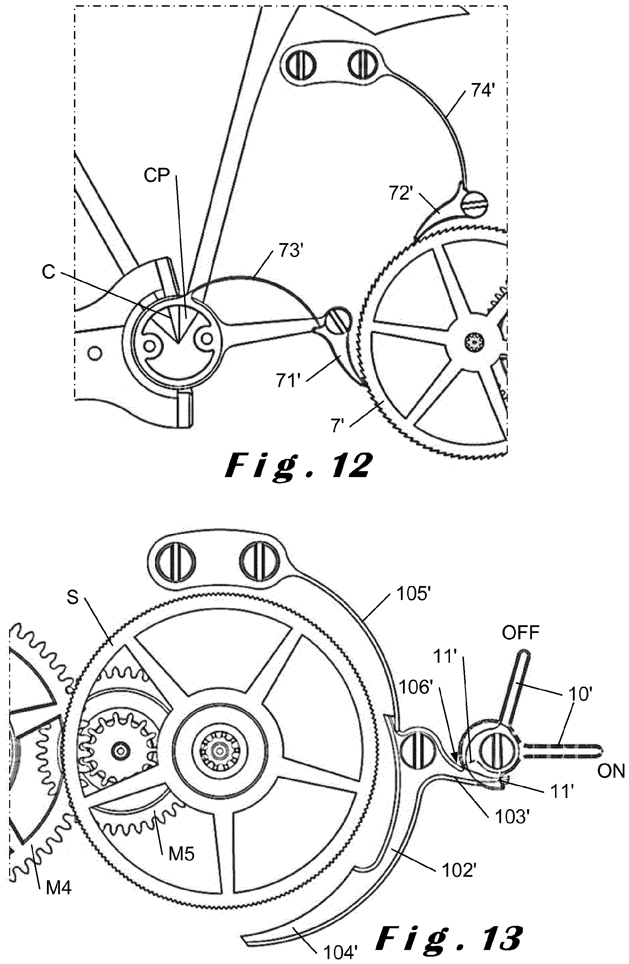

Un dispositif moteur implantable d'un genre alternatif est également représenté aux figures 11 à 13, bien que non couvert par les revendications. Sur la figure 11, ce dispositif moteur implantable est représenté dans une configuration sensiblement linéaire pour simplifier la représentation et la compréhension du lecteur. Cependant cet agencement des éléments constitutifs du mécanisme est purement arbitraire et tout autre agencement plus compact pourrait notamment être choisi. De même, les éléments de protection externe au dispositif ne sont pas représentés sur la figure 11 pour simplifier la représentation. Il est bien évident que les dispositifs mécaniques représentés doivent pour satisfaire son caractère implantable être agencés dans un ou plusieurs boitiers fermés de manière étanche, selon des techniques et matériaux bien connus de l'homme de l'art, en particulier dans le domaine des dispositifs médicaux par exemple.

Ce dispositif moteur implantable 3' comporte lui aussi un mécanisme d'accumulation et de libération séquentielle d'énergie comportant une source d'énergie mécanique 5' agencée en lien cinématique avec, d'une part, un dispositif inertiel de chargement de la source d'énergie mécanique à masse pivotante B sur un pivot à lames P et, d'autre part, au moins un organe d'accouplement, notamment de type prise de force, à un dispositif consommateur tel un implant, une génératrice électromécanique ou autre dispositif requérant la fourniture d'un couple moteur de manière non permanente.

Le mécanisme d'accumulation et de libération séquentielle d'énergie comporte une source d'énergie mécanique 5' formée par un barillet monté pivotant autour d'un arbre, le barillet comportant de façon classique un ressort solidaire à une première extrémité dudit arbre et à une seconde extrémité de la cage 51' du barillet, ladite cage comportant sur sa périphérie extérieure une denture 52' sur laquelle au moins un mobile M5 d'un train de rouage de sortie R2 engrène. Le rouage de sortie R2 peut comporter un nombre variable de mobiles en fonction du rapport de démulplication souhaité à transmettre à un dispositif consommateur. Dans l'exemple représenté, il comporte deux mobiles M4, M5, chacun portant un arbre de sortie constitutant une prise de force vers un dispositif consommateur. Ainsi il est possible de varier le couple de sortie du dispositif moteur implantable 3' simplement par sélection du mobile de sortie M4, M5 auquel accoupler un dispositif consommateur.

Le mécanisme d'accumulation et de libération séquentielle d'énergie comporte un dispositif inertiel de chargement ou remontage du barillet 5'. Ce dispositif inertiel est de type à masse pivotante (ou oscillante) B. De façon originale et caractéristique, la masse pivotante B est agencée mobile en rotation autour d'un axe virtuel parallèle à l'arbre du barillet, ledit axe virtuel étant définit par un dispositif de pivot à lames P, tel que notamment décrit dans la demande WO 2016012281 au nom de la Demanderesse.

Un tel pivot présente l'avantage de présenter des frottements minimaux au niveau de l'axe de rotation de la masse pivotante et donc un rendement maximal. Ce pivot P est constitué d'une ou plusieurs lames s'étendant chacune dans un plan médian commun et dont le fil, c'est-à-dire le tranchant, ou encore le bord de plus faible épaisseur, repose au fond d'une gorge diédrique formant contre-pivot CP. Dans l'exemple représenté, les lames pivot P sont formées et/ou agencées solidaires d'un pont d'ancrage fixe du dispositif et le contre-pivot CP est lui formé solidaire de la masse pivotante B. Toutefois, un agencement inverse des lames pivots P et des contre-pivots CP sur le pont d'ancrage et la masse pivotante peut également être envisagé sans altérer le fonctionnement du dispositif moteur.

L'utilisation d'une masse pivotante B constitue ainsi un organe autonome de remontage du barillet 5', par effet de gravitation, comme dans une montre à remontage automatique ou d'autres mécanismes inertiels d'entrainement. On s'affranchit ainsi d'avoir à connecter le mécanisme remontage à des organes mobiles extérieurs au dispositif moteur 3', ce qui rend celui-ci parfaitement autonome dans la génération de couple moteur dès lors qu'une force d'accélération est appliquée à la masse B, par exemple par déplacement ou mouvement d'un corps ou membre dans lequel le dispositif moteur 3' est implanté.

Pour transmettre un couple de remontage à chaque oscillation de pivotement la masse pivotante B est solidaire ou agencée en liaison cinématique avec un cliquet d'entrainement ou de remontage 71, monté rotatif sur l'extrémité d'un bras de la masse pivotante rappelé par un ressort-lam 73'. Ce cliquet de remontage 71' coopère à chaque alternance avec la denture d'un volant 7' d'entrainement du rochet 6' de remontage du barillet 5', ce volant 7' constituant le mobile d'entrée du mécanisme de remontage du dispositif moteur 3'. Le volant 7' est avantageusement monté rotatif dans un seul sens (en l'espèce antihoraire sur les figures) sur un axe propre et sur lequel est chassé un pignon d'entrainement d'un train de rouages R1, ici constitué de trois roues M1, M2, M3, agencé en liaison cinématique avec la denture du rochet 6' de remontage du du barillet 5', monté solidaire de l'arbre pour remonter le ressort de ce dernier. Afin d'empêcher le recul du rochet 6', un cliquet anti-recul 72' rappelé sur ressort 74' est agencé en relation de la denture du volant 7' d'entrée pour prévenir tout recul du rochet 6' et du rouage R1 dans le sens inverse au sens d'entrainement par le cliquet de remontage 71' sur la masse pivotante B. Des dispositifs de remontage bidirectionnels tels qu'ils en existent dans les montres à remontage automatique pourraient être utilisés également.

Enfin, des moyens de blocage et de libération du barillet 5' en rotation sont également procurés dans le mécanisme d'accumulation et de libération afin de permettre la transmission à la demande du couple mécanique emmagasiné par le ressort du barillet 5' sous l'effet de la masse pivotante B'. Ces moyens de blocage comportent en premier lieu un rateau 102', qui peut être également un levier ou une bascule, mobile en rotation sur un axe ou une goupille, ce rateau 102' comportant un mors 104' de blocage du mobile de sortie S du mécanisme en liaison cinématique avec la denture de barillet 5' via le rouage de sortie R2. Le rateau 102' comporte un bras de pivotement 103' agencé pour coopérer avec un doigt ou loquet de déclenchement 10' rotatif, par exemple sous forme de came ou analogue, agencé pour coopérer avec le bras de pivotement. Le rateau 102' peut etre configuré comme un rateau bloquant, dans lequel cas son mors 104' de blocage du mobile S du rouage de sortie R2, ou un frein, dans lequel cas le mors porte un patin de frottement sur ledit mobile. Dans tous les cas, le mors 104' du rateau 102' est disposé dans une position de « repos » (OFF) du loquet 10' de déclenchement en appui sur la denture dudit mobile de sortie S du train de rouages de sortie R2 du mécanisme. Le loquet 10' comporte un excentrique dont la surface active dans cette position de repos pousse sur le bras de pivotement 103' du rateau 102' pour que le mors de celui-ci reste en contact freinant sur le mobile de sortie S.

Lorsque le loquet 10' est déplacé vers une position d'actionnement « ON », par tout moyen approprié tel qu'une goupille, une came ou autre selon une période de déclenchement déterminée, le loquet 10' libère le bras de pivotement 103' du rateau 102', qui est pivoté dans le sens antihoraire sous l'action d'un ressort de rappel 105'. Le mobile de sortie S est alors libéré de tout verrouillage de même que le rouage de sortie R2, qui entre en rotation sous l'action du couple du ressort du barillet 5' qui libère l'énergie préalablement emmagasinée sous forme de couple moteur disponible sur les prises de forces des mobiles de sortie M5, M6.

Le dispositif moteur des figures 11 à 13 présente l'avantage de procurer une accumulation d'énergie par le barillet 5' permanente et automatique par le balancement gravitaire de la masse pivotante B sur son pivot à lames P. Ce dispositif moteur autonome, de constitution extrêmement simple et fiable permet avantageusement l'actionnement automatique d'un chargeur électrique de type alternateur permettant de recharger de manière autonome par exemple des batteries ou alimenter tout dispositf consommateur, le tout dans un encombrement très réduit.

## Revendications

1. Dispositif moteur implantable (1) comportant une source d'énergie mécanique (5) agencée mobile en rotation autour d'un arbre (4) définissant un axe de rotation (A2) et en liaison cinématique avec, d'une part, un dispositif de chargement de la source d'énergie mécanique, et, d'autre part, un organe d'accouplement à un dispositif consommateur d'énergie, le dispositif de chargement de la source d'énergie mécanique comportant :
- un premier et un second organes de fixation respectivement à un premier et un second membres ou organes mobiles l'un par rapport à l'autre dans le corps d'un patient, agencés tel qu'un déplacement relatif des premier et second membres ou organes entraine un déplacement relatif analogue desdits premier et second organes de fixation ;
- des moyens (6, 7, 8) de remontage de la source d'énergie mécanique (5), lesdits moyens de remontage étant connectés aux dits premier et second organes de fixation et comportant :
i. un rochet (6) solidaire de l'arbre (4) de rotation (A2), ledit rochet (6) comportant une denture périphérique triangulaire, et
ii. un volant (7) d'entrainement unidirectionnel du rochet (6) en rotation avec l'arbre (4), ledit volant (7) portant des cliquets montés mobiles en rotation sur une serge du volant (7) lesdits cliquets comportant une denture complémentaire de celle du rochet de manière à engrener avec la denture périphérique du rochet (6) dans une sens de rotation uniquement lors de chaque déplacement relatif des dits premier et second organes de fixation,
**caractérisé en ce que** le dispositif moteur comporte en outre des moyens (9, 10, 11, 12, 102, 16) de libération séquentielle desdits moyens (6, 7, 8) de remontage de la source d'énergie mécanique (5) pour libérer ladite source d'énergie en rotation afin de transmettre un couple d'entrainement disponible à l'organe d'accouplement, lesdits moyens de libération séquentielle desdits moyens de remontage de la source d'énergie mécanique comportant :
- une came (9) solidaire de la source d'énergie mécanique (5) et
- un cliquet de déclenchement (10) monté mobile en rotation autour d'un axe (A3) parallèle à l'axe (A2) de rotation, une extrémité (101) dudit cliquet (10) étant maintenue en appui par un organe de rappel dans une encoche (91) formée dans une surface de ladite came (9), et
- un doigt de déclenchement (11) solidaire dudit cliquet de déclenchement (10) et agencé pour coopérer avec une goupille (12) solidaire du rochet (6) dans une position unique dudit rochet de manière à pivoter ledit cliquet de déclenchement (10) de telle sorte que son extrémité (101) sorte de l'encoche (91) de la came et libère la source d'énergie mécanique (5) en rotation pour transmettre un couple d'entrainement à l'organe d'accouplement,
- un levier de blocage (102) solidaire en rotation du cliquet de déclenchement (10) et agencé pour coopérer en butée avec un doigt de blocage (16) lorsque l'extrémité (101) du cliquet de déclenchement est insérée dans l'encoche (91).

2. Dispositif moteur implantable selon la revendication 1, dans lequel la source d'énergie mécanique est un barillet (5), doté d'une cage (51) munie d'une denture périphérique (52) externe d'une part et logeant un ressort de barillet d'autre part, ledit ressort étant solidaire d'une paroi interne de la cage en une première extrémité et de l'arbre de rotation (4) en une seconde extrémité,

3. Dispositif moteur implantable selon la revendication 1 ou 2, **caractérisé en ce que** le rochet (6) est monté libre en rotation par rapport au volant (7) par l'intermédiaire d'un roulement à billes (8).

4. Dispositif moteur implantable selon l'une des revendications 1 à 3, **caractérisé en ce qu'**il comporte en outre des cliquets de blocage (73) du rochet (6), lesdits cliquets de blocage (73) comportant une denture complémentaire de celle du rochet (6) configurée pour empêcher une rotation du rochet en sens opposé à celui d'entrainement par le volant (7).

5. Dispositif moteur implantable selon l'une des revendications 1 à 4, dans lequel un train de rouages (14) est agencé en prise entre le mécanisme d'accumulation et de libération séquentielle d'énergie mécanique et l'organe d'accouplement à un dispositif consommateur.

6. Dispositif moteur implantable selon la revendication 5, dans lequel le train de rouages (14) est un train multiplicateur.

7. Dispositif moteur implantable selon l'une des revendications 1 à 6, dans lequel un doigt (16) de blocage des moyens de libération séquentielle est agencé pour coopérer en butée avec le levier (102) du cliquet de déclenchement (10) lorsque ce dernier est inséré dans l'encoche (91) de la came (9).

8. Dispositif moteur implantable selon la revendication 7, dans lequel le levier (102) du cliquet de dégagement (10) comporte un profil (103) de dégagement du doigt (16) de blocage, agencé pour permettre une libération sensiblement instantanée du barillet (5) en rotation dès la sortie du cliquet de déclenchement (10) de l'encoche de la came (9) lors de la levée du doigt de déclenchement (11) par la goupille (12).

9. Dispositif moteur implantable selon l'une des revendications 1 à 8, dans lequel l'organe d'accouplement est choisi parmi un mobile denté, une prise de force, un mobile d'entrainement magnétique.

10. Dispositif moteur implantable selon l'une des revendications 1 à 9, dans lequel le mécanisme (3) d'accumulation et de libération séquentielle d'énergie est agencé dans un boitier inoxydable et étanche.

11. Dispositif électro-générateur implantable, comportant un dispositif moteur implantable selon l'une des revendications 1 à 10 et un générateur électromécanique (2) comportant un rotor accouplé à l'organe d'accouplement du dispositif moteur.

12. Dispositif électro-générateur implantable selon la revendication 11, comportant en outre un dispositif (17) d'accumulation d'énergie électrique générée par le générateur électromécanique (2), électriquement connecté à une sortie dudit générateur (2).

13. Dispositif électro-générateur implantable selon l'une des revendications 11 ou 12, comportant en outre des moyens électroniques de gestion de la distribution du courant délivré par le générateur (2).

14. Dispositif électro-générateur implantable selon l'une des revendications 11 à 13, dans lequel le générateur électromécanique (2) et le dispositif moteur sont agencés dans un boitier étanche.

15. Dispositif électro-générateur implantable selon l'une des revendications 13 ou 14, dans lequel des moyens de connexion électrique étanches sont agencés sur le boitier.

## Patentansprüche

1. Implantierbare Motorvorrichtung (1), umfassend eine mechanische Energiequelle (5), die drehbeweglich um eine Welle (4) angeordnet ist, die eine Drehachse (A2) definiert und kinematisch mit einerseits einer Vorrichtung zum Laden der mechanischen Energiequelle und andererseits einem Teil zum Koppeln an eine energieverbrauchende Vorrichtung verbunden ist, wobei die Vorrichtung zum Laden der mechanischen Energiequelle umfasst:
- ein erstes Teil und ein zweites Teil zum Befestigen an einem ersten Element oder Teil beziehungsweise einem zweiten Element oder Teil, die relativ zueinander in einem Körper eines Patienten beweglich sind und derart angeordnet sind, dass eine relative Verschiebung des ersten Elements oder Teils und des zweiten Elements oder Teils eine analoge relative Verschiebung des ersten und des zweiten Befestigungsteils bewirkt;
- Mittel (6, 7, 8) zum Aufziehen der mechanischen Energiequelle (5), wobei die Aufziehmittel mit dem ersten und dem zweiten Befestigungsteil verbunden sind und umfassen:
i. ein Sperrrad (6), das einstückig mit der Welle (4) für die Drehung (A2) ist, wobei das Sperrrad (6) eine Umfangsdreiecksverzahnung umfasst, und
ii. ein Schwungrad (7) zum unidirektionalen Antreiben des Sperrrads (6) in Drehung mit der Welle (4), wobei das Schwungrad (7) drehbewegliche Klinken auf einem Rand des Schwungrads (7) trägt, wobei die Klinken Zähne umfassen, die komplementär zu denen des Sperrrads sind, um in die Umfangsverzahnung des Sperrrads (6) in nur einer Drehrichtung bei jeder relativen Verschiebung des ersten und des zweiten Befestigungsteils einzugreifen,
**dadurch gekennzeichnet, dass** die Motorvorrichtung ferner Mittel (9, 10, 11, 12, 102, 16) zum sequentiellen Freigeben der Mittel (6, 7, 8) zum Aufziehen der mechanischen Energiequelle (5) umfasst, um die Energiequelle zur Drehung freizugeben, um ein verfügbares Antriebsmoment auf das Kopplungsteil zu übertragen, wobei die Mittel zum sequentiellen Freigeben der Aufziehmittel der mechanischen Energiequelle umfassen:
- einen Nocken (9), der einstückig mit der mechanischen Energiequelle (5) ist, und
- eine Freigabeklinke (10), die um eine Achse (A3), die parallel zur Drehachse (A2) ist, drehbeweglich gelagert ist, wobei ein Ende (101) der Klinke (10) durch ein Rückstellelement in einer Einbuchtung (91), die in einer Oberfläche des Nockens (9) ausgebildet ist, in Anlage gehalten wird, und
- einen Freigabefinger (11), der einstückig mit der Freigabeklinke (10) und dazu eingerichtet ist, mit einem mit dem Sperrrad (6) einstückigen Zapfen (12) in einer einzigen Position des Sperrrads zusammenzuwirken, um die Freigabeklinke (10) derart zu schwenken, dass ihr Ende (101) aus der Einbuchtung (91) des Nockens austritt und die mechanische Energiequelle (5) zur Drehung freigibt, um ein Antriebsmoment auf das Kopplungsteil zu übertragen,
- einen Verriegelungshebel (102), der mit der Freigabeklinke (10) drehfest verbunden und dazu eingerichtet ist, in Anlage mit einem Verriegelungsfinger (16) zusammenzuwirken, wenn das Ende (101) der Freigabeklinke in die Einbuchtung (91) eingefügt ist.

2. Implantierbare Motorvorrichtung nach Anspruch 1, wobei die mechanische Energiequelle eine Trommel (5) ist, die mit einem Käfig (51) ausgestattet ist, der einerseits mit einer äußeren Umfangsverzahnung (52) versehen ist und andererseits eine Tonnenfeder beherbergt, wobei die Feder an einem ersten Ende mit einer Innenwand des Käfigs und an einem zweiten Ende mit der Drehwelle (4) fest verbunden ist.

3. Implantierbare Motorvorrichtung nach Anspruch 1 oder 2, **dadurch gekennzeichnet, dass** das Sperrrad (6) mithilfe eines Kugellagers (8) gegenüber dem Schwungrad (7) frei drehbar gelagert ist.

4. Implantierbare Motorvorrichtung nach einem der Ansprüche 1 bis 3, **dadurch gekennzeichnet, dass** sie ferner Klinken (73) zum Sperren des Sperrrads (6) umfasst, wobei diese Sperrklinken (73) Zähne umfassen, die komplementär zu denen des Sperrrads (6) sind und dazu eingerichtet sind, eine Drehung des Sperrrads in der zur Antriebsrichtung entgegengesetzten Richtung durch das Schwungrad (7) zu verhindern.

5. Implantierbare Motorvorrichtung nach einem der Ansprüche 1 bis 4, wobei ein Zahnradgetriebe (14) in Eingriff zwischen dem Mechanismus zur Speicherung und sequentiellen ,Freisetzung von mechanischer Energie und dem Teil zur Kopplung mit einer Verbrauchervorrichtung angeordnet ist.

6. Implantierbare Motorvorrichtung nach Anspruch 5, wobei das Zahnradgetriebe (14) ein Übersetzungsgetriebe ist.

7. Implantierbare Motorvorrichtung nach einem der Ansprüche 1 bis 6, wobei ein Finger (16) zum Verriegeln der Mittel für die sequentielle Freisetzung dazu eingerichtet ist, in Anlage mit dem Hebel (102) der Freigabeklinke (10) zusammenzuwirken, wenn letztere in die Einbuchtung (91) des Nockens (9) eingefügt ist.

8. Implantierbare Motorvorrichtung nach Anspruch 7, wobei der Hebel (102) der Freigabeklinke (10) ein Profil (103) zum Lösen des Verriegelungsfingers (16) umfasst, das dazu eingerichtet ist, eine im Wesentlichen sofortige Freigabe der Trommel (5) zur Drehung zu ermöglichen, sobald die Freigabeklinke (10) aus der Einbuchtung in dem Nocken (9) austritt, wenn der Freigabefinger (11) durch den Zapfen (12) angehoben wird.

9. Implantierbare Motorvorrichtung nach einem der Ansprüche 1 bis 8, wobei das Kopplungsteil aus einem Zahnrad und Ritzel, einer Zapfwelle oder einem Magnettriebrad und Ritzel ausgewählt ist.

10. Implantierbare Motorvorrichtung nach einem der Ansprüche 1 bis 9, wobei der Mechanismus (3) zur Speicherung und sequentiellen Freisetzung von Energie in einem nicht oxidierenden und dichten Gehäuse angeordnet ist.

11. Implantierbare Stromerzeugungsvorrichtung, umfassend eine implantierbare Motorvorrichtung nach einem der Ansprüche 1 bis 10 und einen elektromechanischen Generator (2), der einen Rotor umfasst, der mit dem Kopplungsteil der Motorvorrichtung gekoppelt ist.

12. Implantierbare Stromerzeugungsvorrichtung nach Anspruch 11, ferner umfassend eine Vorrichtung (17) zum Speichern von elektrischer Energie, die von dem elektromechanischen Generator (2) erzeugt wird, die mit einem Ausgang des Generators (2) elektrisch verbunden ist.

13. Implantierbare Stromerzeugungsvorrichtung nach einem der Ansprüche 11 oder 12, ferner umfassend elektronische Mittel zum Verwalten und Verteilen des von dem Generator (2) gelieferten Stroms.

14. Implantierbare Stromerzeugungsvorrichtung nach einem der Ansprüche 11 bis 13, wobei der elektromechanische Generator (2) und die Motorvorrichtung in einem dichten Gehäuse angeordnet sind.

15. Implantierbare Stromerzeugungsvorrichtung nach einem der Ansprüche 13 oder 14, wobei dichte elektrische Verbindungsmittel auf dem Gehäuse angeordnet sind.

## Claims

1. An implantable motor device (1) comprising a mechanical energy source (5) arranged rotationally mobile about a shaft (4) which defines an axis of rotation (A2) and is kinematically linked with, on the one hand, a device for charging the mechanical energy source, and, on the other hand, a member for coupling to an energy-consuming device, the device for charging the mechanical energy source comprising:
- a first member and a second member for attachment respectively to a first element or member and a second element or member which are mobile in relation to one another in a patient's body and arranged such that relative displacement of the first and second elements or members brings about an analogous relative displacement of said first and second attachment members;
- means (6, 7, 8) for winding the mechanical energy source (5), said winding means being connected to said first and second attachment members and comprising:
i. a ratchet (6) integral with the shaft (4) of rotation (A2), said ratchet (6) comprising triangular peripheral teeth, and
ii. a flywheel (7) for unidirectionally driving the ratchet (6) in rotation with the shaft (4), said flywheel (7) bearing pawls mobile in rotation on a rim of the flywheel (7) said pawls comprising teeth complementary to those of the ratchet so as to mesh with the peripheral teeth of the ratchet (6) in just one direction of rotation on each relative displacement of said first and second attachment members,
**characterised in that** the motor device furthermore comprises means (9, 10, 11, 12, 102, 16) for sequentially liberating said means (6, 7, 8) for winding the mechanical energy source (5) to liberate said energy source in rotation in order to transmit an available driving torque to the coupling member, said means for sequentially liberating said winding means of the mechanical energy source comprising:
- a cam (9) integral with the mechanical energy source (5) and
- a release pawl (10) mounted rotationally mobile about an axis (A3) parallel to the axis (A2) of rotation, one end (101) of said pawl (10) being held in abutment by a return member in a notch (91) formed in a surface of said cam (9), and
- a release finger (11) integral with said release pawl (10) and arranged to cooperate with a pin (12) integral with the ratchet (6) in a single position of said ratchet so as to pivot said release pawl (10) in such a manner that its end (101) exits from the notch (91) of the cam and liberates the mechanical energy source (5) in rotation to transmit a drive torque to the coupling member,
- a locking lever (102) rotationally integral with the release pawl (10) and arranged to cooperate in abutment with a locking finger (16) when the end (101) of the release pawl is inserted in the notch (91) .

2. An implantable motor device according to Claim 1, in which the mechanical energy source is a barrel (5) equipped with a cage (51) provided with external peripheral teeth (52) on the one hand and accommodating a barrel spring on the other hand, said spring being integral with an inner wall of the cage at a first end and with the rotation shaft (4) at a second end.

3. An implantable motor device according to Claim 1 or 2, **characterised in that** the ratchet (6) is freely rotationally mounted in relation to the flywheel (7) by means of a ball bearing (8).

4. An implantable motor device according to one of Claims 1 to 3, **characterised in that** it further comprises pawls (73) for locking the ratchet (6), said locking pawls (73) comprising teeth complementary to those of the ratchet (6) and configured to prevent rotation of the ratchet in the opposite direction to the direction of drive by the flywheel (7).

5. An implantable motor device according to one of Claims 1 to 4, in which a geartrain (14) is arranged in engagement between the mechanism for storage and sequential liberation of mechanical energy and the member for coupling to a consumer device.

6. An implantable motor device according to Claim 5, in which the geartrain (14) is a multiplier train.

7. An implantable motor device according to one of Claims 1 to 6, in which a finger (16) for locking the sequential liberation means is arranged to cooperate in abutment with the lever (102) of the release pawl (10) when the latter is inserted in the notch (91) of the cam (9).

8. An implantable motor device according to Claim 7, in which the lever (102) of the release pawl (10) comprises a profile (103) for disengaging the locking finger (16), arranged to permit substantially instantaneous liberation of the barrel (5) in rotation as soon as the release pawl (10) exits from the notch in the cam (9) when the release finger (11) is lifted by the pin (12).

9. An implantable motor device according to one of Claims 1 to 8, in which the coupling member is selected from among a toothed wheel and pinion, a power take-off or a magnetic drive wheel and pinion.

10. An implantable motor device according to one of Claims 1 to 9, in which the energy storage and sequential liberation mechanism (3) is arranged in a non-oxidising and leaktight casing.

11. An implantable electrical generation device comprising an implantable motor device according to one of Claims 1 to 10 and an electromechanical generator (2) comprising a rotor coupled to the coupling member of the motor device.

12. An implantable electrical generation device according to Claim 11, further comprising a device (17) for storing electrical energy generated by the electromechanical generator (2) and electrically connected to an output of said generator (2).

13. An implantable electrical generation device according to one of Claims 11 or 12, further comprising electronic means for managing and distributing the current supplied by the generator (2).

14. An implantable electrical generation device according to one of Claims 11 to 13, in which the electromechanical generator (2) and the motor device are arranged in a leaktight casing.

15. An implantable electrical generation device according to one of Claims 13 or 14, in which leaktight electrical connection means are arranged on the casing.
